# EUROPEAN PATENT SPECIFICATION

(11) **EP 1 668 091 B1**
(45) Date of publication and mention of the grant of the patent: **26.03.2008**
(21) Application number: 04768710.8
(22) Date of filing: 30.09.2004
(51) Int. Cl.: C09K 11/06, C07D 209/12, G01N 33/58

(54) **FLUORESCENT COMPOUND**
FLUORESZIERENDE VERBINDUNG
COMPOSE FLUORESCENT

(30) Priority: 03.10.2003 GB 0323171
(43) Date of publication of application: 14.06.2006
(73) Proprietor: COVENTRY UNIVERSITY, Coventry CV1 5FB (GB)
(72) Inventor: LYNCH, Daniel Eric, Coventry CV1 5FB (GB); HEPTINSTALL, John, Coventry CV1 5FB (GB)
(74) Representative: Chapple, Colin Richard
(86) International application number: PCT/GB2004/004167
(87) International publication number: WO 2005/033245

(56) References cited:
- WO-A-01/02374
- WO-A-01/11370
- US-A1- 2002 147 354
- TERPETSCHNIG E ET AL: "SYNTHESIS, SPECTRAL PROPERTIES AND PHOTOSTABILITIES OF SYMMETRICAL AND UNSYMMETRICAQL SQUARAINES; A NEW CLASS OF FLUOROPHORES WITH LONG-WAVELENGTH EXCITATION AND EMISSION" ANALYTICA CHIMICA ACTA, ELSEVIER, AMSTERDAM, NL, vol. 282, no. 3, 29 October 1993 (1993-10-29), pages 633-641, XP000563718 ISSN: 0003-2670 cited in the application

## Description

The present invention relates to novel photoluminescent compounds, to a novel protein detector and to a novel method of detecting the presence of protein in a fluid, in particular water, or bodily fluids.

Photoluminescent compounds are known, and known photoluminescent compounds include the class of dyes known as squaraine dyes, in particular the indolenine series of squaraine dyes, which are substituted and unsubstituted compounds of the general formula:

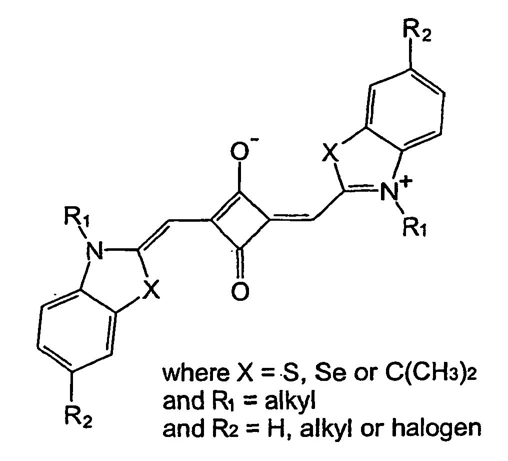

Known indolenine squaraine dyes include both symmetrical and asymmetrical compounds, and are described, for example, in an article by E. Terpetschnig et al. in Anal. Chim. Acta 282 (1993) pages 633 - 641.

These dyes are insoluble in water and are used, inter alia, for the qualitative detection of the presence of protein in fluids. In order to use these dyes for the detection of the presence of proteins, which are themselves water-soluble in a fluid, in particular an aqueous fluid, the dye has to be dissolved in a solvent comprising a mixture of water and an alcohol such as methanol. When protein is added to a water/methanol solution of an indolenine squaraine dye, there is an increase in fluorescence. The analytical method disclosed in the article cited above is a purely qualitative method, simply recording the presence or absence of protein in the test fluid sample, and cannot therefore be used to determine the amount of protein present.

Current methods for the quantitative analysis of protein in fluids and semiquantitatively / quantitatively as stains for proteins in gels include the Bradford [M.M. Bradford in Anal. Biochem. 72 (1976) 248], Lowry [O.H. Lowry et al. in J. Biol. Chem. 193 (1951) 265] methods and bicinchoninic acid (BCA) [P.K. Smith et al. in Anal. Biochem.150 (1985) 76] methods for fluids and silver stain [C.R Merril in Meth. Enzymol. 182 (1990) 477] and Coomassie Blue [S. Fazekas de St. Groth et al. in Biochim. Biophys. Acta 71 (1963) 377] for gels. The Bradford, Lowry and BCA methods are colourimetric techinques (ie. colour change) and are suitable for solution protein ranges of 8 - 2000 µg/mL, require multiple reagents and incubation times and suffer from interference from numerous common reagents. Coomassie Blue, used in the Bradford method, takes 20 - 30 mins and is not hindered by tris buffers. Silver stain is a very sensitive (10 - 100 ng/mL) chromatogenic-technique and is used as a gel stain but care must be taken in the handling of the gel and the whole technique takes 2 - 3 hours. Coomassie Blue is also a gel stain and can be used instead of silver stain if the protein range is in the chromatogenic µg range. Other commercial techniques for the detection of protein concentration include the NanoOrange^{™} Protein Quantitation Kit and the CBQCA^{™} Protein Quantitation Kit, both from Molecular Probes, which are ultra-sensitive solution techniques reliant on changes in fluorescence but are not linear over the ranges given for the squaraine derivative. These two techniques also require 30 mins preparation time.

It is an object of the present invention to provide a novel photoluminescent compound, in particular a novel photoluminescent compound that is soluble in water, or water containing solvent mixtures, and retains its photoluminescent properties in these media.

It is a further obj ect of the present invention to provide a method for detecting the presence of protein in fluids, or as a gel stain, in which the above time constraints are reduced or substantially obviated and at a greater sensitivity.

The present invention provides water-soluble photoluminescent compounds including a symmetrical skeletal structure of the formula 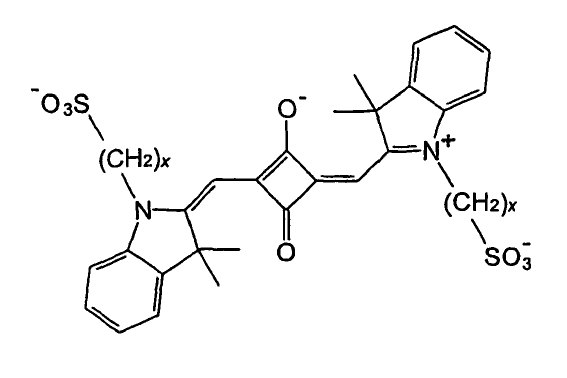 in which x may represent any integer, and in which the phenyl rings may be substituted.

Particularly preferred compounds according to the invention include those wherein x = 3.

Further preferred compounds according to the invention include those which are unsubstituted or in which one or both phenyl rings are substituted in the 5-position with alkyl (including ⁱpropyl and ^{t}butyl) or halogen groups.

Particularly preferred compounds according to the invention include 2,4-bis(1-(propan-3-sulfonic acid)-3,3-trimethyl-2-indolinylidenemethyl)cyclobutenediylium-1,3-diolate or any metal or quaternary nitrogen (ie. ammonium, mono-, di- or trialkylammonium, pyridinium etc) salt of the sulfonic acid.

The present invention further provides a protein detector, which comprises a compound including a symmetrical skeletal structure of the general formula 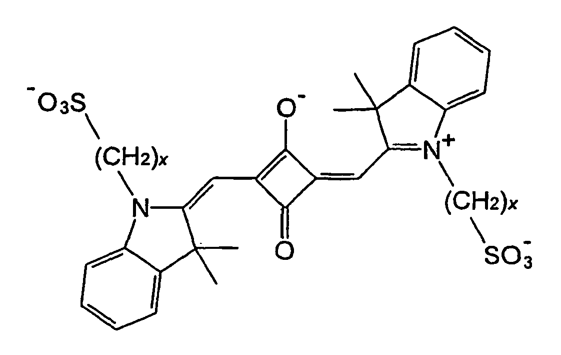 in which x may represent any integer, and in which the phenyl rings may be substituted, in solution in water or a water containing solvent mixture, at concentrations from 1 x 10⁻⁹ to 1 moles per litre.

The present invention further provides a method for measuring the total dissolved protein content of a fluid sample, which method includes the steps of:
(a) dissolving a compound including a symmetrical skeletal structure of the general formula; 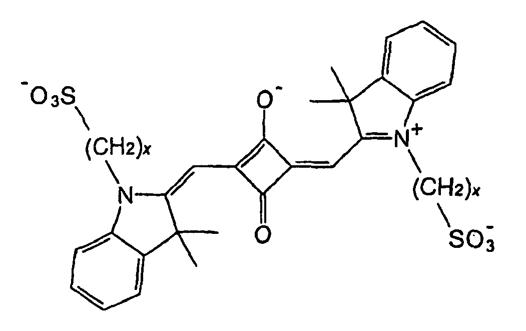 in which x may represent any integer, and in which the phenyl rings may be substituted, in aqueous solution at concentrations from 1 x 10⁻¹⁰ to 1 moles per litre.
(b) admixing the solution of step (a) with a test fluid sample;
(c) measuring the fluorescence of the sample; and
(d) comparing the fluorescence with a standard value or values (ie. calibration plot), to obtain a value for the total protein content.

The present invention further provides a method for detecting and / or quantifying proteins separated electrophoretically in a supporting matrix, for example polyacrylamide, agrose or starch, either in the presence or absence of sodium dodecylsulfate (SDS), that has been fixed in an aqueous / organic / acid mixture comprising aqueous methanol and acetic acid, wherein the matrix is subsequently stained with a photoluminescent compound including a symmetrical skeletal structure of the general formula; 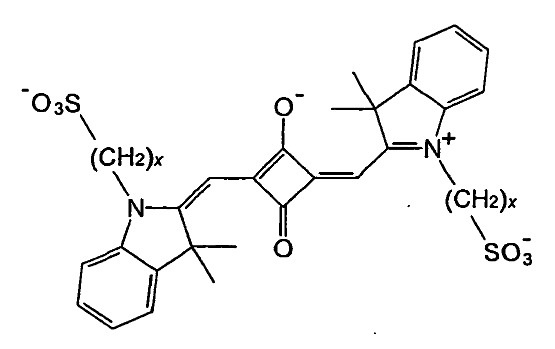 in which x may represent any integer, and in which the phenyl rings may be substituted, in solution in 10% aqueous methanol or aqueous acetic acid, at a concentration of from 1 x 10⁻¹⁰ to 1 moles per litre, and destained to visualise bands.

In order to establish standard values for the fluorescence of aqueous solutions of proteins over the required detection ranges, the fluorescence of solutions containing known concentrations of the protein BSA, was measured using a 5 x 10⁻⁸ M aqueous solution of the compound including a symmetrical skeletal structure of formula: 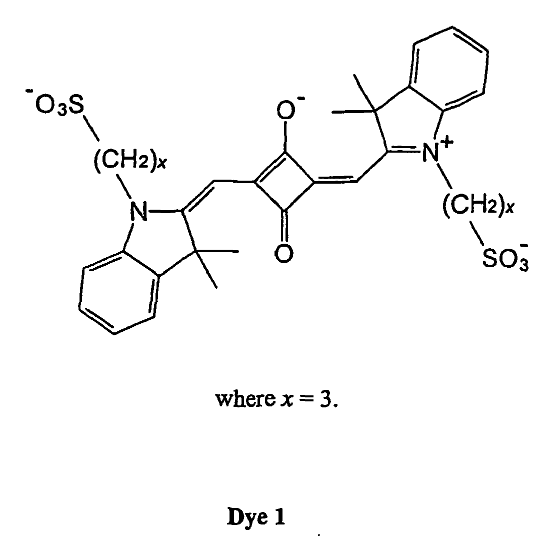 at an excitation wavelength of 624 nm (ε =1.0 x 10⁵ mol⁻¹cm⁻¹) and a detection wavelength of 638 nm.

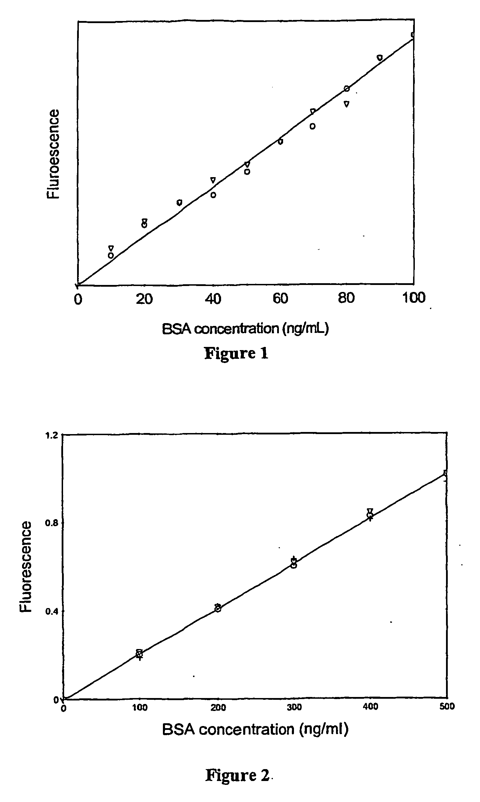

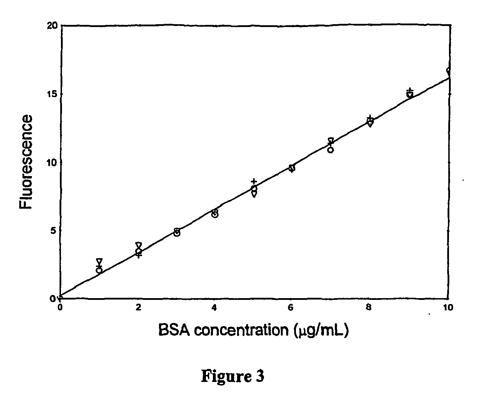

The results, for two separate calibration plots over the range 0-100 ng/mL BSA are shown in Figure 1; the results for three separate calibration plots, over the detection range of 0-500 ng/mL BSA are shown in Figure 2 and the results, for three separate calibration plots, over the detection range of 0-10 µg/mL BSA are shown in Figure 3.

It is particularly striking to note the linear response over these detection ranges.

It is also an advantage of the photo luminescent compounds of the present invention, that once a calibration curve has been constructed using BSA then any number of protein concentrations can be determined instantaneously. The fluorescence is also at a high wavelength and is not masked by any diffuse lower wavelength organic fluorescence.

### Experimental

### Starting materials

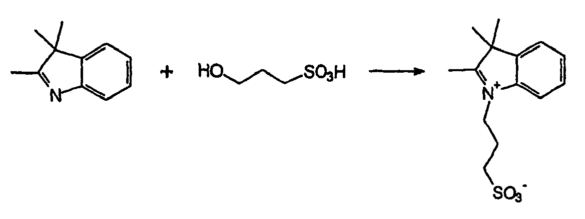

2,3,3-trimethyl-1-(propan-3-sulfonyl)indolenine was prepared using the literature method of E. Havinga et al. in Synthetic Metals 69 (1995) pages 581-582 by heating 2,3,3-trimethylindolenine in excess 1,3-propanesultone and toluene, using a Dean and Stark apparatus. Upon cooling the excess solvent was decanted off and the product washed repeatedly with petroleum ether to yield a red oil. Crystals of 2,3,3-trimethyl-1-(propan-3-sulfonyl)indolenine formed from the oil upon standing for several weeks and the single crystal x-ray structure is shown in Figure 4.

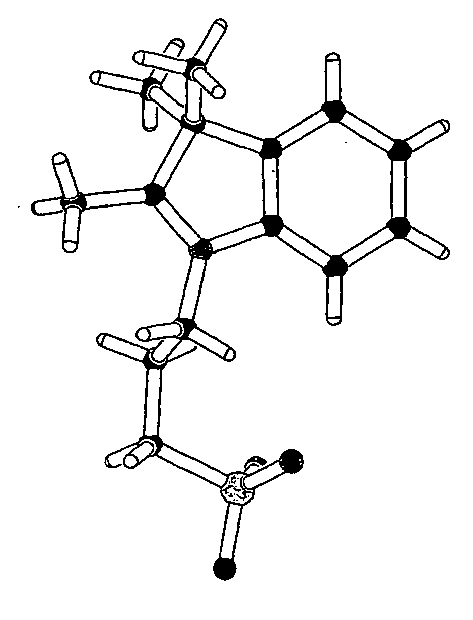

### Figure 4

### Molecular conformation and atom-naming scheme for the structure of 2,3,3-trirnethyl-1-(propan-3-sulfonyl)indolenine.

### Preparation of Dye 1

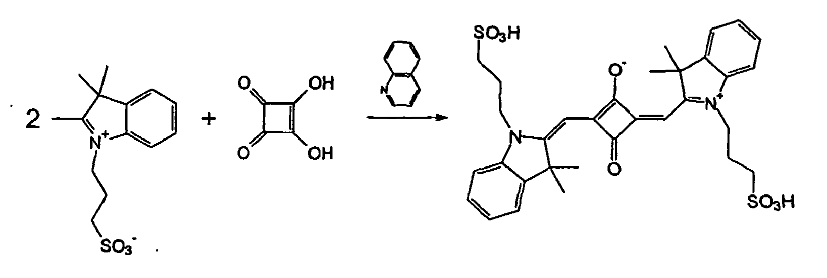

2,4-bis(1-(propan-3-sulfonic acid)-3,3-dimethyl-2-indolinylidenemethyl)cyclobutenebis(ylium)-1 3-diolate was prepared using the literature method of Sprenger and Ziegenbein in Agnew. Chem. Int. Ed. 6 (1967) page 533 by refluxing 2:1 molar amounts of 2,3,3-trimethyl-1-(propan-3-sulfonyl)indolenine and squaric acid with catalytic amounts of quinaline in 50/50 toluene/butan-1-ol using a Dean and Stark apparatus. The product was collected in vacuo after removal of the reaction solvents and repeated heated washings with petroleum ether.

## Claims

1. A water-soluble photoluminescent compound including a symmetrical skeletal structure of the formula 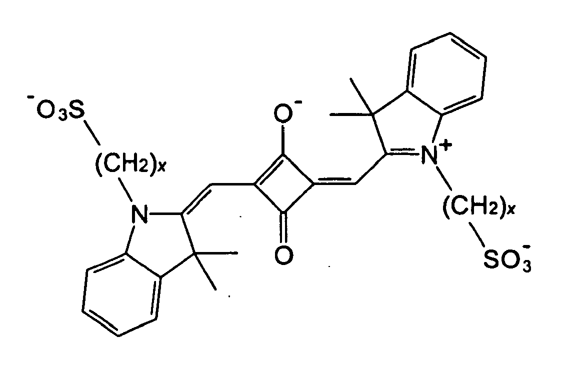 in which x may represent any integer, and in which the phenyl rings may be substituted.

2. A photoluminescent compound according to claim 1 wherein x = 3.

3. A photoluminescent compound according to claim 1 or claim 2 which is unsubstituted.

4. A photoluminescent compound according to claim 1 or claim 2 in which one or both phenyl rings are substituted in the 5-position with alkyl, including ⁱpropyl and ^{t}butyl, or halogen groups.

5. A photoluminescent compound according to any of claims 1 to 4 which is 2,4-bis(1-(propan-3-sulfonic acid)-3,3-dimethyl-2-indolinylidenemethyl)cyclobutenediylium-1,3-diolate.

6. A photoluminescent compound according to any of claims 1 to 4 which is selected from any metal or quaternary nitrogen salt of the sulfonic acid

7. A photoluminescent compound according to any of claims 1 to 4 which is selected from ammonium, mono-, di- or trialkylammonium, pyridinium salt of the sulfonic acid

8. A protein detector, which comprises a photoluminescent compound including a symmetrical skeletal satructure of the general formula 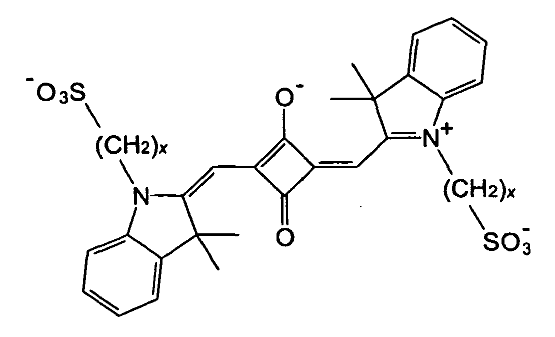 in which x may represent any integer, and in which the phenyl rings may be substituted, in solution in water, at concentrations from 1 x 10⁻¹⁰ to 1 moles per litre.

9. A method for measuring the total dissolved protein content of a fluid sample, which method includes the steps of:
(a) dissolving a photoluminescent compound including a symmetrical skeletal structure of the general formula; 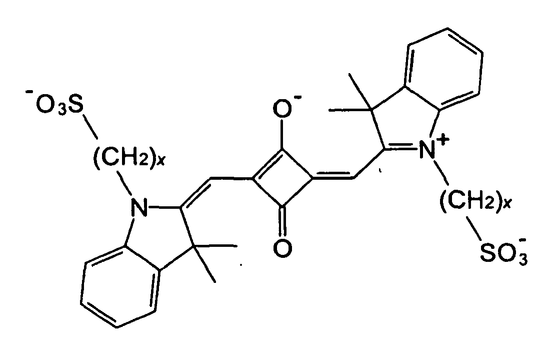 in which x may represent any integer, and in which the phenyl rings may be substituted, in solution in water, at concentrations from 1 x 10⁻¹⁰ to 1 moles per litre.
(b) admixing the solution of step (a) with a test fluid sample;
(c) measuring the fluorescence of the sample; and
(d) comparing the fluorescence with a standard value, to obtain a value for the total dissolved protein content.

10. A method for detecting and / or quantifying proteins separated electrophoretically in a supporting matrix, either in the presence or absence of sodium dodecylsulfate (SDS), that has been fixed in an aqueous / organic / acid mixture comprising aqueous methanol and acetic acid, wherein the matrix is subsequently stained with a photoluminescent compound including a symmetrical skeletal structure of the general formula; 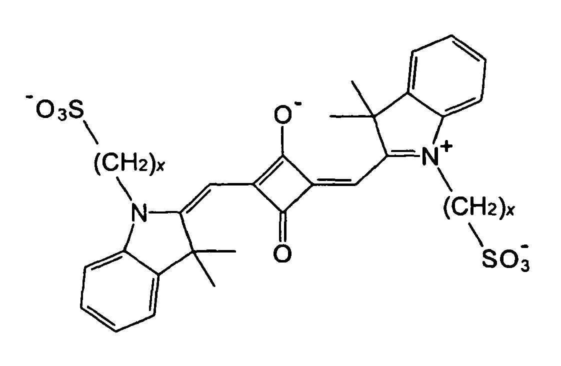 in which x may represent any integer, and in which the phenyl rings may be substituted, in solution in 10% aqueous methanol or aqueous acetic acid, at a concentration of from 1 x 10⁻¹⁰ to 1 moles per litre, and destained to visualise bands.

11. A method according to claim 10 wherein the supporting matrix is polyacrylamide, agarose or starch.

## Patentansprüche

1. Wasserlösliche photolumineszierende Verbindung umfassend eine symmetrische Skelettstruktur der Formel: 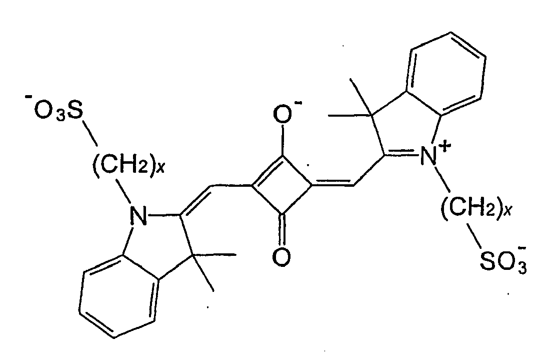 wobei x irgendeine ganze Zahl darstellen kann und wobei die Phenylringe substituiert sein können.

2. Photolumineszierende Verbindung nach Anspruch 1, wobei x = 3 ist.

3. Photolumineszierende Verbindung nach Anspruch 1 oder Anspruch 2, die unsubstituiert ist.

4. Photolumineszierende Verbindung nach Anspruch 1 oder Anspruch 2, wobei ein oder beide Phenylringe in Position 5 mit Alkyl, einschließlich ¹Propyl und ¹Butyl, oder Halogengruppen substituiert ist bzw. sind.

5. Photolumineszierende Verbindung nach einem der Ansprüche 1 bis 4, die 2,4-Bis(1-(propan-3-sulfonsäure)-3,3-dimethyl-2-indolinylidenmethyl)cyclobutendiylium-1,3-diolat ist.

6. Photolumineszierende Verbindung nach Anspruch 5, die unter irgendeinem Metall- oder quaternären Stickstoffsalz der Propylsulfonsäuregruppe ausgewählt wird.

7. Photolumineszierende Verbindung nach Anspruch 6, die unter Ammonium, Mono-, Di- oder Trialkylammonium-, Pyridiniumsalz der Propylsulfonsäuregruppe ausgewählt ist.

8. Proteindetektor, der eine photolumineszierende Verbindung umfassend eine symmetrische Skelettstruktur der allgemeinen Formel: 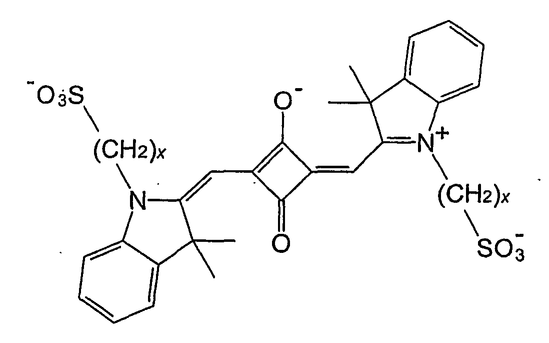 wobei x irgendeine ganze Zahl darstellen kann und wobei die Phenylringe substituiert sein können, in Lösung in Wasser in einer Konzentration von 1 x 10⁻¹⁰ bis 1 Mol pro Liter umfasst.

9. Methode zum Messen des gesamten Gehalts an gelöstem Protein einer Flüssigkeitsprobe, welche Methode folgende Schritte umfasst:
(a) Lösen einer photolumineszierenden Verbindung umfassend eine symmetrische Skelettstruktur der allgemeinen Formel: 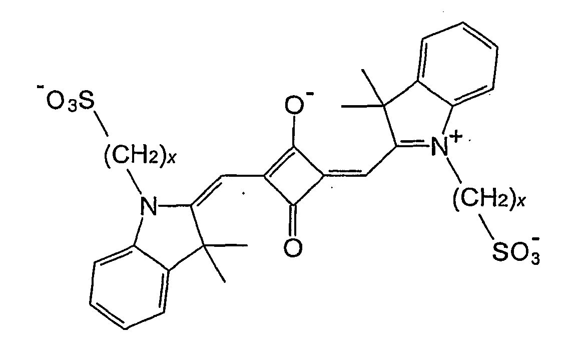 wobei x irgendeine ganze Zahl darstellen kann und wobei die Phenylringe substituiert sein können, in einer wässrigen Lösung in einer Konzentration von 1 x 10-¹⁰ bis 1 Mol pro Liter;
(b) Mischen der Lösung aus Schritt (a) mit einer Testflüssigkeitsprobe;
(c) Messen der Fluoreszenz der Probe; und
(d) Vergleichen der Fluoreszenz mit einem Standardwert, um einen Wert für den gesamten Gehalt an gelöstem Protein zu erhalten.

10. Methode für das Erfassen und/oder Quantifizieren von Proteinen, die in einer Trägermatrix elektrophoretisch getrennt sind, entweder in Gegenwart oder Abwesenheit von Natriumdodecylsulfat (SDS), das in einer Mischung fixiert worden ist, die wässriges Methanol und Essigsäure umfasst, wobei die Matrix daraufhin mit einer photolumineszierenden Verbindung gefärbt wird umfassend eine symmetrische Skelettstruktur der allgemeinen Formel: 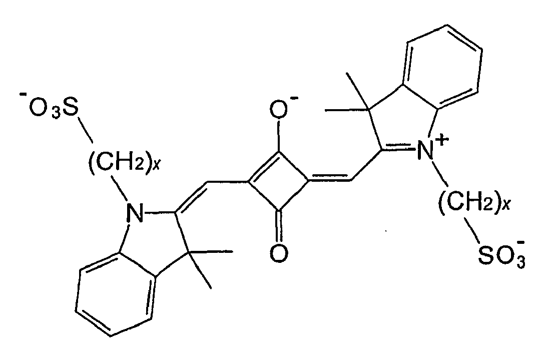 wobei x irgendeine ganze Zahl darstellen kann und wobei die Phenylringe substituiert sein können, in Lösung in 10% wässrigem Methanol oder wässriger Essigsäure in einer Konzentration von 1 x 10⁻¹⁰ bis 1 Mol pro Liter und zum Sichtbarmachen von Banden entfärbt wird.

11. Methode nach Anspruch 9, wobei die Stützmatrix Polyacrylamid, Agarose oder Stärke ist.

## Revendications

1. Composé photoluminescent hydrosoluble incluant une structure squelettique symétrique de formule 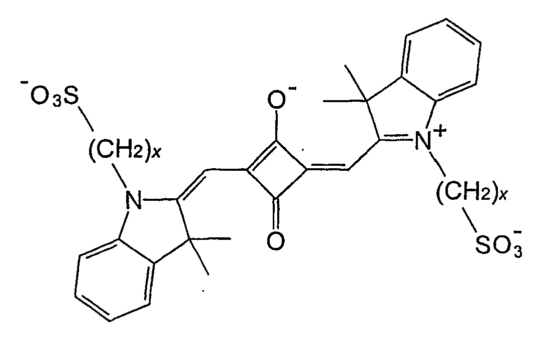 dans laquelle x peut représenter tout entier, et dans laquelle les cycles phényles peuvent être substitués.

2. Composé photoluminescent selon la revendication 1, dans lequel x = 3.

3. Composé photoluminescent selon la revendication 1 ou la revendication 2, qui est non substitué.

4. Composé photoluminescent selon la revendication 1 ou la revendication 2, dans lequel l'un des cycles phényles, ou les deux, est/sont substitué(s) en position 5 par alkyle, incluant ⁱpropyle et ^{t}butyle, ou des groupes halogénés.

5. Composé photoluminescent selon l'une quelconque des revendications 1 à 4, qui est le cyclobutènediylium-1,3-diolate de 2,4-bis(1-(acide propan-3-sulfonique)-3,3-diméthyl-2-indolinylidèneméthyle).

6. Composé photoluminescent selon la revendication 5, qui est choisi parmi tout sel de métal ou d'azote quaternaire du groupe acide propyl sulfonique.

7. Composé photoluminescent selon la revendication 6, qui est choisi parmi un sel d'ammonium, de mono-, di- ou trialkylammonium, de pyridinium du groupe acide propyl sulfonique.

8. Détecteur de protéine, qui comprend un composé photoluminescent incluant une structure squelettique symétrique de formule générale 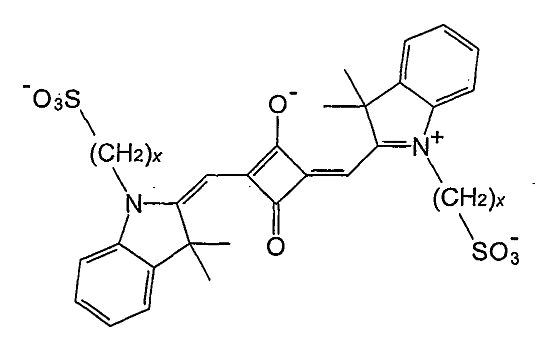 dans laquelle x peut représenter tout entier, et dans laquelle les cycles phényles peuvent être substitués, en solution dans de l'eau, à des concentrations de 1 x 10⁻¹⁰ à 1 mole par litre.

9. Procédé pour doser la teneur en protéine dissoute totale d'un échantillon fluide, lequel procédé inclut les étapes consistant:
(a) à dissoudre un composé photoluminescent incluant une structure squelettique symétrique de formule générale; 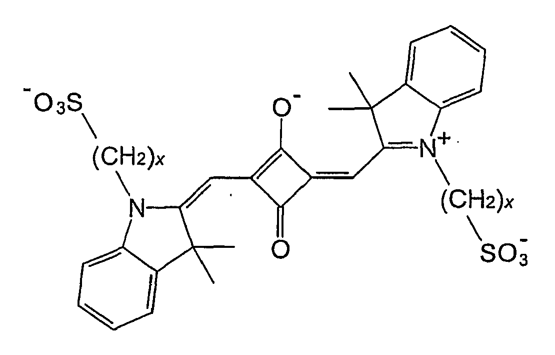 dans laquelle x peut représenter tout entier, et dans laquelle les cycles phényles peuvent être substitués, dans une solution aqueuse, à des concentrations de 1 x 10⁻¹⁰ à 1 mole par litre.
(b) à mélanger la solution de l'étape (a) avec un échantillon d'essai fluide;
(c) à mesurer la fluorescence de l'échantillon; et
(d) à comparer la fluorescence avec une valeur étalon, pour obtenir une valeur pour la teneur en protéine dissoute totale.

10. Procédé pour détecter et/ou quantifier des protéines séparées électrophorétiquement dans une matrice support, soit en présence soit en l'absence de dodécylsulfate de sodium (SDS), qui a été fixée dans un mélange incluant méthanol et acide acétique aqueux, dans lequel la matrice est ensuite colorée avec un composé photoluminescent incluant une structure squelettique symétrique de formule générale; 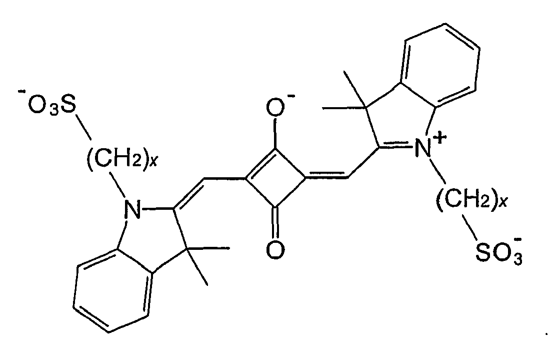 dans laquelle x peut représenter tout entier, et dans laquelle les cycles phényles peuvent être substitués, en solution dans méthanol aqueux ou acide acétique aqueux à 10%, à une concentration de 1 x 10⁻¹⁰ à 1 mole par litre, et décolorée pour visualiser les bandes.

11. Procédé selon la revendication 9, dans lequel la matrice support est un polyacrylamide, de l'agarose ou de l'amidon.
